(19) **Europäisches Patentamt**

**European Patent Office**

**Office européen des brevets**

(11) Veröffentlichungsnummer : **0 373 380 B1**

(12) # EUROPÄISCHE PATENTSCHRIFT

(45) Veröffentlichungstag der Patentschrift :
**11.11.92 Patentblatt 92/46**

(21) Anmeldenummer : **89121167.4**

(22) Anmeldetag : **16.11.89**

(51) Int. Cl.⁵ : **A61K 6/04,** A61C 5/10,
A61C 13/10

(54) **Verfahren zur Herstellung eines gesinterten Zahnersatzes.**

(30) Priorität : **13.12.88 DE 3841902**

(43) Veröffentlichungstag der Anmeldung :
**20.06.90 Patentblatt 90/25**

(45) Bekanntmachung des Hinweises auf die
Patenterteilung :
**11.11.92 Patentblatt 92/46**

(84) Benannte Vertragsstaaten :
**AT BE CH DE ES FR GB GR IT LI LU NL SE**

(56) Entgegenhaltungen :
**EP-A- 0 214 341**
**DE-C- 3 717 048**
**FR-A- 2 036 946**

(73) Patentinhaber : **Degussa Aktiengesellschaft**
**Weissfrauenstrasse 9**
**W-6000 Frankfurt am Main 1 (DE)**

(72) Erfinder : **Klaus, Angela**
**Feldstrasse 9**
**W-6450 Hanau 8 (DE)**
Erfinder : **Groll, Werner, Dr., Dipl.-Ing.**
**Gartenstrasse 5**
**W-8755 Alzenau-Hörstein (DE)**
Erfinder : **Lange, Thomas**
**Gerauer Strasse 86 A**
**W-6000 Frankfurt/M. 71 (DE)**

## Beschreibung

Die Erfindung betrifft ein Verfahren zur Herstellung eines gesinterten Zahnersatzes mit metallischer Gefügematrix aus einer Edelmetalloder Edelmetallegierungspulvermischung mit bi- oder mehrmodaler Teilchengrößenverteilung und vorwiegend kugeliger Gestalt, die mit einer im wesentlichen aus Wasser bestehenden Anmischflüssigkeit zu einem modellierfähigen und durch Austreiben der Anmischflüssigkeit verdichtbaren Schlicker angerührt wird, mit dem der Zahnersatz auf einem als Brennträger dienenden Modell der zu versorgenden Zähne mit der bei Dentalkeramik üblichen Technik modelliert, und anschließend auf dem Modell in einer Graphitbox oder unter Schutzgas gesintert wird.

Die Herstellung metallischen Zahnersatzes zur prothetischen Versorgung bei Zahnerkrankungen bzw. nach Verlust eines oder mehrerer Zähne, wie z.B. Inlays, mit Keramik oder Kunststoff verblendbare oder nicht verblendete Kronen und Brücken, erfolgt üblicherweise mit den sogenannten "Wachsausschmelzverfahren", einer Feingußtechnik, die hohe Maßhaltigkeit gewährleistet.

Die Vorteile der so hergestellten Kronen und Brücken sind neben der Maßhaltigkeit vor allem in der hohen Festigkeit und der vorhandenen Dukitilität zu sehen, die bei größeren Brückenkonstruktionen zur Vermeidung von Gewaltbrüchen bei Überbelastung gewährleistet sein müssen. Dagegen ist das Verfahren selbst sehr zeitraubend, material- und geräteintensiv. Die Notendigkeit der Verwendung von Angußkanälen und Gußkegel verursacht einen gegenüber dem Gewicht des Gußobjektes deutlich erhöhten Materialeinsatz, der bei mehrmaliger Wiederverwendung zu Veränderungen der Legierungseigenschaften führen kann, und - falls er nicht wiederverwendet wird - als Schrott zurückbleibt. Ein weiterer Nachteil dieser Technik ist, daß bei Fehlern im Gußobjekt eine Reparatur nicht möglich ist, sondern der gesamte Herstellungsprozeß, beginnend bei der Wachsmodellation, wiederholt werden muß.

Die DE-OS 1 915 977 beschreibt ein Verfahren zur sintertechnologischen Herstellung von metallischem Zahnersatz, bei dem mit einer Paste, die aus Metallpulver mit einer Partikelgröße zwischen 2 und 25 μm und einem als Kleber fungierenden Bindemittel besteht, der Zahnersatz auf einem Modell der zu versorgenden Zähne modelliert und anschließend gesintert wird. Der Nachteil des Verfahrens liegt in der schlechten Verdichtbarkeit der beschriebenen Pasten, da der als Kleber wirkende Binder nicht durch Verdichtungsverfahren, wie Riffeln oder Rütteln, ausgetrieben werden kann. Da weiterhin von einer Pulverfraktion ausgegangen wird, ist die Dichte des Grünlings gering. Dies hat zur Folge, daß beim Sintern eine sehr große Schrumpfung und eine demzufolge nicht tolerierbare Paßungenauigkeit auftritt. Die Verwendung von sehr feinen Pulvern zwischen 2 und 25 μm gewährleistet zwar eine sehr hohe Sinteraktivität, verursacht aber zusätzlich hohe Herstellungskosten.

Das in der US-Patentschrift 46 61 071 beschriebene Verfahren zur sintertechnologischen Herstellung von metallischem Zahnersatz benutzt Pulver mit einer Größe von 5-90 μm - angeteigt mit einem geeigneten Binder - für die Modellation des metallischen Zahnersatzes auf einem Modell der zu versorgenden Zähne. Für die Herstellung des Modells ist eine spezielle, gießbare und selbst erhärtende Stumpfmasse notwendig, die vor dem Auftragen des Metallpulvers bei 1400° C - 1460° C gebrannt werden muß.

Da herkömmliche Dentalkeramikaufbrennöfen Maximaltemperaturen bis ca. 1200° C erreichen, ist hierfür ein Spezialofen erforderlich. Zum Sintern des Metallpulvers wird ein Flüssigphasensinterprozeß unter einem Vakuum von 1 HPa bis $10^{-2}$ HPa verwendet. Da herkömmliche Dentalkeramik-Aufbrennöfen dieses Vakuum nicht erreichen, ist ebenfalls ein spezieller Vakuumofen nötig. Öfen mit Maximaltemperaturen bis 1400° C und Öfen, die ein gutes Vakuum bei hohen Temperaturen gewährleisten, sind sehr viel teurer als normale Keramik-Aufbrennöfen, so daß die Anwendung dieser Verfahren beim Zahntechniker eine teure Geräteinvestition erfordert. Die Verwendung des Flüssigphasensinterprozesses führt außerdem zu Problemen bezüglich der Formstabilität während des Sinterns. Um eine möglichst schnelle Verdichtung durch eine Umordnung der festen Bestandteile (Partikeln) zu erhalten, ist ein Flüssigphasenanteil von mindestens 30 - 35 % notwendig. (R.M. German, Liquid Phase Sintering, Plenum Press, N.Y.) Seiten 4,6,80). Analog dem Verhalten von Dentalaufbrennkeramik ist mit einer Abrundung bzw. Abflachung von sehr grazilen Details, z.B. einer Okklusalfläche zu rechnen, was zu Problemen bezüglich der Kontaktpunkte führen kann und unter Umständen eine erhebliche Nachbearbeitung erfordert.

In der DE-OS 35 32 331 wird ein Verfahren zur sintertechnologischen Herstellung von metallischem Zahnersatz beschrieben. das unter Verwendung einer Pulvermischung mit mehrmodaler Größenverteilung, die mit Wasser in einen modellierfähigen und verdichtbaren Schlicker überführt wird, eine gezielt hohe Dichte des Grünlings erreicht und dementsprechend die Schrumpfung während des Sinterns gering bleibt. Dies ist für die Erzielung einer guten Paßgenauigkeit vorteilhaft. Die Verwendung von Wasser als Anmischflüssigkeit und einer Konsistenz, die der von Dentalverblendkeramikschlickern sehr ähnlich ist, erlaubt eine zusätzliche Verdichtung durch Austreiben der Flüssigkeit mit der bei Dentalkeramik üblichen Technik (Riffeln etc.). Der Sinterprozeß ist ohne größeren Aufwand in einem herkömmlichen Dentalkeramik-Aufbrennofen durchführbar.

Dies kann einerseits durch Verwendung einer Graphitbox erreicht werden, in der sich der modellierte und zu sinternde Zahnersatz befindet. Diese Graphitbox wird in einen üblichen Dentalkeramik-Aufbrennofen gestellt und gewährleistet bei der Sintertemperatur einen Schutz gegen die Oxidation von Nichtedelmetallbestandteilen der Legierung. Andererseits kann durch Einleiten von Schutzgas in den Keramik-Aufbrennofen ebenfalls eine ausreichende Reduzierung des Sauerstoffpartialdruckes erreicht werden. Nach dem Sintern wird der Zahnersatz in der Graphitbox an Luft abgekühlt.

Als Nachteile des Verfahrens bei Verwendung von Pulvermischungen aus verdüsten, vorwiegend kugeligen Edelmetall-Legierungen und gefällten, sehr feinen, vorwiegend kugeligen Edelmetallpulvern stellte sich heraus, daß mit den dort beschriebenen Sinterparametern keine maximalen Dichtewerte im gesinterten Zustand erreichbar sind und daß insbesondere bei mehrfachem Sintern die Dichte des gesinterten Zahnersatzes deutlich abfällt. Mehrfaches Sintern kann aber notwendig sein bei der Herstellung von Brücken in mehreren Arbeitsschritten oder bei Randkorrekturen.

Es war daher Aufgabe der vorliegenden Erfindung, ein Verfahren gemäß dem Oberbegriff von Patent Anspruch 1 zu entwickeln, mit dem optimale Dichtwerte im gesinterten Zustand zu erreichen sind, insbesondere beim Mehrfachsintern, unter Verwendung gebräuchlicher Geräte und Techniken.

Diese Aufgabe wird mit den Maßnahmen des kennzeichnenden Teils des Patentanspruchs 1 gelöst.

Vorteilhafte Ausgestaltungen des Verfahrens zur Herstellung von gesintertem Zahnersatz ergeben sich aus den Maßnahmen der Unteransprüche.

Der modellierte und verdichtete Zahnersatz wird nach einer Trocknung an der Luft (5 bis 25 min.) beispielsweise in eine Graphitbox gestellt und in einem Temperaturbereich zwischen 100° und 400° C 5-45 min wärmebehandelt. Danach wird mit einer mittleren Temperaturerhöhung von 50 bis 300 K bis auf 800° C und mit 20 bis 200 K/min an Luft oder unter Schutzgas von der Vorwärmtemperatur auf die Sintertemperatur T erhitzt, die zwischen ($T_{solidus}$ -200° C) und ($T_{solidus}$ -70° C) liegt, wobei $T_{solidus}$ die Solidustemperatur der gesinterten Legierung ist. Bei dieser Temperatur wird 5 bis 45 min an Luft oder unter Schutzgas gesintert und anschließend im Temperaturbereich unterhalb 900° C unter einem Vakuum von 1 - 50 HPa oder unter Schutzgas abgekühlt. Nach der Abkühlung kann der Zahnersatz der Graphitbox entnommen werden.

Vorzugsweise wird das Aufheizen auf Sintertemperatur und das Sintern in der Graphitbox an Luft, die Abkühlung im Temperaturbereich unterhalb 900° C unter Vakuum durchgeführt. Dies ist insbesondere im Hinblick auf die Verwendung von im Dentallabor üblicherweise vorhandenen Geräten und bezüglich der Kosten von Vorteil.

Bevorzugte Zeiten für die Wärmebehandlung zwischen 100° C und 400° C und das Sintern liegen bei 5 - 25 min bzw. 10-30 min.

Bei Verzicht auf das Arbeiten in einer Graphitbox muß unter Schutzgas gearbeitet werden, wobei der Sauerstoffpartialdruck weniger als 5 x $10^{-2}$ HPa betragen sollte. Dies ist z.B. bei Verwendung von Argon mit technischer Reinheit gewährleistet. Durch relativ einfache Umbauten eines gebräuchlichen Keramikofens ist dies realisierbar.

Ein besonderer Vorteil dieses Verfahrens ist, daß mit den genannten Parametern - insbesondere der Vakuumkühlung - auch nach mehrfachem Sintern eine ausreichend hohe Dichte, gepaart mit geschlossener Porosität, erreicht wird.

Bei dem erfindungsgemäßen Verfahren wird eine Mischung aus vorwiegend kugelförmigen Pulvern mit bi- oder mehrmodaler Verteilung verwendet. Diese Pulvermischung wird mit einer Anmischflüssigkeit, die vorwiegend aus Wasser besteht, aber auch geringe Zusätze von Elektrolyten, wie z.B. Strontiumchlorid, Kupferchlorid oder Ammoniumnitrat, ein- oder mehrwertige Alkohole, Cellulose oder Polyethylenglykol enthalten kann, zu einem Schlicker mit Hand oder mit einem hierfür geeigneten Rührer angemischt, dessen Konsistenz und Modellationseigenschaften denen von üblicher Dental- bzw. Verblendkeramik entsprechen.

Der so präparierte Schlicker wird mit der bei Dentalkeramik üblichen Technik auf ein hochtemperaturfestes Modell der zu versorgenden Zähne aufgetragen und dort durch bekannte Techniken (z.B. Rütteln mit dem Riffelteil eines Modellierinstrumentes, Ultraschall etc.) verdichtet. Dabei tritt die Flüssigkeit an die Oberfläche, die mit einem Tuch abgesaugt oder im warmen Luftstrom getrocknet wird. Vor dem Auftragen des Schlickers empfiehlt es sich, den Stumpf mit Flüssigkeit zu tränken oder zu isolieren, damit dem Schlicker die Feuchtigkeit nicht vom Stumpf entzogen wird.

Der zu hoher Rohdichte verdichtete Grünling wird zunächst an Luft auf dem Modell ca. 5-25 min stehen gelassen, um langsam abzutrocknen. Dies kann auch auf der Deckplatte eines Keramikaufbrennofens geschehen, der normalerweise eine Temperatur $\leqq$ 50°C aufweist. Danach wird der auf dem Modell befindliche Zahnersatz in eine Graphitbox gegeben, die ihn vollständig umschließt.

Der Zahnersatz wird dann in einem Ofen bei einer Temperatur zwischen 100 und 400° C 5-45 Minuten wärmebehandelt. Diese Wärmebehandlung dient dazu, evtl. noch vorhandene Feuchtigkeit oder organische Verunreinigungen zu entfernen. Führt man diese Wärmebehandlung vor dem eigentlichen Sintern nicht durch, so kommt es zu Rißbildung in den Kronenwänden. Überschreitet man die Temperaturen von 400° C und hält die Temperatur entsprechend den angegebenen Zeiten, so kommt es zu einem drastischen Abfall der Dichte des gesinterten Zahnersatzes.

Nach der Wärmebehandlung wird die Graphitbox mit dem darin befindlichen Zahnersatz auf die Sintertemperatur aufgeheizt. Dabei ist es für das Erreichen einer ausreichend hohen Sinterdichte notwendig, den Temperaturbereich zwischen 400° C und 800° C mit einer mittleren Aufheizgeschwindigkeit von mehr als 50 K/min zu überbrücken, insbesondere mit 50 bis 300 K/min.

Kleinere Aufheizgeschwindigkeiten führen zu einer reduzierten Dichte. Oberhalb 800° C wird die mittlere Aufheizgeschwindigkeit günstigerweise zwischen 20 und 200 K/min gewählt, was auch im Hinblick auf die gesamte Sinterdauer zu vertretbaren Zeiten führt.

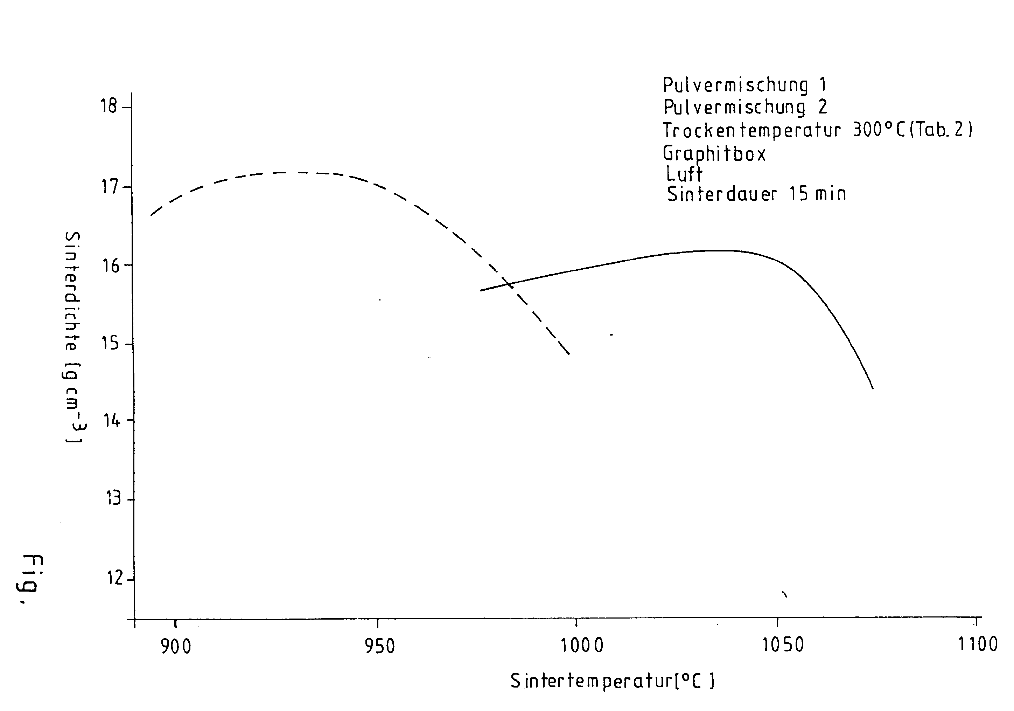

Die Dichte des gesinterten Zahnersatzes ist abhängig von der Sintertemperatur T. Überraschenderweise hat sich nun gezeigt, daß die Dichte in einem Temperaturbereich zwischen ($T_{solidus}$ -200° C) und ($T_{solidus}$ -70° C) ein Maximum aufweist, wobei die genaue Lage wiederum von der speziellen Legierung abhängt. Das Maximum erstreckt sich über einen Temperaturbereich von 20-50° C, wobei nach Überschreitung des Maximums ein sehr starker Abfall der Dichtewerte beobachtet wird. Zwei typische Kurven für zwei Pulvermischungen sind in der Abbildung zu sehen.

Das Sintern des Zahnersatzes in der Graphitbox kann an Luft oder unter Schutzgas durchgeführt werden. Vorzugsweise wird an Luft gesintert, da die erhaltenen Ergebnisse nicht schlechter sind als unter Schutzgas und der apparative Aufwand vergleichsweise geringer ist.

Die Sinterdauer beträgt 5-45 min, wobei im Regelfall bei Sinterzeiten zwischen 10 und 30 min bereits die maximale Dichte erreicht wird.

Eine Abkühlung des gesinterten Zahnersatzes in der Graphitbox an Luft führt zwar nach dem ersten Sinterschritt zu hohen Dichten. Für die Herstellung von Brücken oder zur Korrektur der Okklusion, der Kontaktpunkte oder des Randschlusses muß jedoch ggf. ein zweiter Sinterschritt angeschlossen werden. Trotz gleichen Sinterzyklusses nimmt die Dichte des gesinterten Zahnersatzes bei zweifachem Sintern drastisch ab.

Überraschenderweise hat sich nun gezeigt, daß durch Abkühlung des in der Graphitbox befindlichen Zahnersatzes unter einem Vakuum zwischen 50 und 1 HPa der Dichteabfall bei mehrfachem Sintern verhindert werden kann. Entscheidend ist, daß das geforderte Vakuum vorhanden ist, bevor der Zahnersatz auf Temperatur < 900° C abgekühlt ist.

Es ist auch ein Sintern ohne Graphitbox möglich, jedoch muß dann das Sintern unter Schutzgas erfolgen, wobei die vorgenannten Temperatur- und Zeitangaben ebenso gelten. Der Sauerstoffpartialdruck in Schutzgas darf zur Erzielung einer ausreichend hohen Dichte 5 x 10⁻² HPa nicht überschreiten. Die Abkühlung muß dann ebenfalls unter Schutzgas erfolgen.

Folgende Beispiele sollen das erfindungsgemäße Verfahren näher erläutern:

1. Der Schlicker wird aus der Pulvermischung 1 (Tab.2), durch Zugabe einer Anmischflüssigkeit aus 98 % $H_2O$ und 2 % Polyethylenglykol in eine zum Modellieren geeignete Konsistenz überführt. Der Schlicker wird nun mit einem Pinsel auf einen mit Flüssigkeit getränkten Stumpf aufgetragen. Die Krone wird vollständig modelliert und die Form immer wieder im Artikulator überprüft.

Durch Riffeln mit dem Modellierinstrument wird der Schlicker verdichtet. Die aus der Oberfläche austretende Flüssigkeit wird mit einem Tuch abgesaugt. Die gute Standfestigkeit des Schlickers erlaubt den Aufbau von Details der Okklusalfläche, wie Höcker oder Gräben. Nach der Verdichtung (es tritt keine Feuchtigkeit mehr aus der Oberfläche aus) kann die Oberfläche durch Schaben oder Schnitzen nachgearbeitet werden, so daß auch feine Fissuren vor dem Sintern geschaffen werden können. Die fertig modellierte Krone bleibt während des gesamten Sinterprozesses auf dem Stumpf. Zum Trocknen wird sie auf die Deckplatte eines Keramikaufbrennofens gestellt und nach 15 min in eine Graphitbox gegeben. Die Graphitbox besteht aus einem Graphitboden mit entsprechender Aufnahme für den Stumpf und einem becherförmigen Graphitdeckel. Die Graphitbox mit der modellierten Krone wird in einen Ofen gestellt, der gleichzeitig auf 300° C aufgeheizt wird. Nach 15 min wird die Graphitbox in einen auf 1000° C vorgeheizten Keramikaufbrennofen gegeben und die Temperatur auf 1050° C erhöht. Die Sintertemperatur von 1050°C wird nach 5 Minuten erreicht, was einer mittleren Aufheizgeschwindigkeit von 150 K/min entspricht. Die Sintertemperatur von 1050° C liegt 160° C unter der $T_{solidus}$ - Temperatur von 1210° C (siehe Tabelle 2). Nach 20 Minuten wird die Graphitbox aus dem Ofen genommen und kühlt an Luft ab. An der Okklusalfläche und in einem Randbereich sind einige kleinere Korrekturen vorzunehmen. Auf der gesinterten Krone werden anschließend wie oben beschrieben die zu korrigierenden Stellen angetragen. Danach wird die Krone unter Verwendung des oben bereits beschriebenen Sinterzyklus erneut gesintert. Die Überprüfung der Dichte ergibt einen Wert von 14,2 g/cm³. Die Krone ist zu groß, weil die Abkühlung an der Luft erfolgte.

2. Analog Beispiel 1 wird eine weitere Krone hergestellt. Nach Abschluß des Sinterns bei 1050° C wird die Graphitbox mit der Krone jedoch in eine evakuierbare Abkühlkammer umgesetzt. Unmittelbar nach Umsetzen der Graphitbox wird die Abkühlkammer auf ein Vakuum von ca. 50 HPa evakuiert. Die abgekühlte Probe kann nach ca. 15 min. entnommen werden. Wie in Beispiel 1 beschrieben, werden einige kleinere Korrekturen durchgeführt. Die Krone wird erneut gesintert und wiederum unter Vakuum abgekühlt. Die Stumpfmasse wird unter Verwendung eines Sandstrahlgerätes ausgestrahlt und die Dichte bestimmt. Sie beträgt nunmehr 16,1 g/cm³, die Porosität ist geschlossen. Die Krone wird ausgearbeitet und poliert. Der Randspalt auf dem Meistermodell beträgt im Mittel 40 μm.

   3. Zur Herstellung von Inlays wird die Pulvermischung 2 (Tabelle 2) verwendet, da diese Legierung gelb ist und von vielen Patienten bevorzugt wird. Außerdem besitzt diese eine geringere 0,2 % - Dehngrenze und eine erhöhte Duktilität. Dies ermöglicht ein leichteres Anfinieren der Ränder im Mund. Die Herstellung verläuft analog dem unter Beispiel 2 beschriebenen Verfahren.

Der Sinterzyklus wird jedoch etwas geändert. Nach der Trocknung an Luft wird das auf dem Modellstumpf befindliche Inlay (in Graphitbox) auf den Brenntisch eines auf 700° C vorgeheizten geöffneten Keramik-Aufbrennofens gestellt. Auf den Brenntisch herrscht eine Temperatur von ca. 250°C. Nach 9 min fährt der Brenntisch automatisch ein und der Ofen heizt auf die Sintertemperatur von 940° C auf. Nach weiteren 15 min kann die Probe aus dem Ofen entnommen, in die Abkühlkammer umgesetzt und dort unter Vakuum 50 HPa abgekühlt werden. Die Stumpfmasse wird ausgestrahlt, das Inlay ausgearbeitet, auf dem Meistermodellstumpf aufgesetzt und poliert. Die Dichte beträgt 17,1 g/cm³, die Porosität ist geschlossen. Durch die Politur werden auch die an der Oberfläche gelegenen Poren geschlossen. Der Randspalt beträgt ca. 50 um. Die Temperaturänderungsgeschwindigkeit zwischen 400 und 800°C betrug hierbei im Mittel etwa 120 K/min, die Temperaturänderungsgeschwindigkeit zwischen 800° C und der Sintertemperatur im Mittel 100 K/min. $T_{solidus}$ = 1040° C (Tabelle 2).

Auf die gleiche Weise lassen sich auch Palladium-Legierungen verarbeiten.

Die Tabelle 1 gibt die Zusammensetzung der in den Beispielen verwendeten Legierungen, deren Herstellung, Kornform und Korngröße an, während Tabelle 2 die Zusammensetzung der in den Beispielen verwendeten Pulvermischungen zeigt.

Die Abbildung stellt die Abhängigkeit der Sinterdichte von Zahnersatzteilen gemäß der in Tabelle 2 enthaltenen Pulvermischungen von der Sintertemperatur dar.

Tabelle 1: Beispiele für eingesetzte Pulver

| | Zusammensetzung (Massenanteil in %) | Kornform | Herstellung | Korngröße/$\mu$m |
|---|---|---|---|---|
| Legierung 1 | Au 65 Pt 15 Pd 13 In 2,5 + Zusätze je < 2 % | vorwiegend kugelig | Verdüsung | –* |
| Legierung 2 | Au 87 Pt 11 + Zusätze je < 2 % | vorwiegend kugelig | Verdüsung | –* |
| Au-Pulver 1 | Au | " | chem.Fällung | < 5 |
| Au-Pulver 2 | Au | " | chem.Fällung | < 10 |

* je nach Aussiebung, stets < 100 $\mu$m

EP 0 373 380 B1

Tabelle 2:

| | Komponente 1 Pulver Gehalt KG**/$\mu$m % | | | Komponente 2 Pulver Gehalt KG/$\mu$m % | | | Komponente 3 Pulver Gehalt KG/$\mu$m % | | | $T_{solidus}$* |
|---|---|---|---|---|---|---|---|---|---|---|
| Pulvermischg.1 | Leg. 1 | 80 | < 63 | Au 1 | 20 | < 5 | | | | 1210$^{o}$C |
| Pulvermischg.2 | Leg. 2 | 85 | < 50 | Au 1 | 1.3 | < 5 | Au 2 | 2 | < 10 | 1040$^{o}$C |

* $T_{solidus}$ der gesinterten Legieurng

** KG = Korngröße

EP 0 373 380 B1

EP 0 373 380 B1

**Patentansprüche**

1. Verfahren zur Herstellung eines gesinterten Zahnersatzes mit metallischer Gefügematrix aus einer Edelmetall- oder Edelmetallegierungspulvermischung mit bi- oder mehrmodaler Teilchengrößenverteilung und vorwiegend kugeliger Gestalt, die mit einer im wesentlichen aus Wasser bestehenden Anmischflüssigkeit zu einem modellierfähigen und durch Austreiben der Anmischflüssigkeit verdichtbaren Schlicker angerührt wird, mit dem der Zahnersatz auf einem als Brennträger dienenden Modell der zu versorgenden Zähne mit der bei Dentalkeramik üblichen Technik modelliert und anschließend auf dem Modell in einer Graphitbox oder unter Schutzgas gesintert wird,
dadurch gekennzeichnet,
daß der modellierte und zunächst an Luft 5 bis 25 min getrocknete Zahnersatz 5 bis 45 min zwischen 100° C und 400° C wärmebehandelt, danach auf 800° C mit einer mittleren Temperaturerhöhung von 50 bis 300 K/min erhitzt und oberhalb 800° C mit einer mittleren Temperaturerhöhung von 20 bis 200 K/min an Luft (in der Graphitbox) oder unter einem Schutzgas auf die Sintertemperatur T gebracht wird, die zwischen ($T_{solidus}$ -200° C) und ($T_{solidus}$ -70° C) liegt, wobei $T_{solidus}$ die Solidustemperatur der gesinterten Legierung ist, bei dieser Temperatur 5 bis 45 min an der Luft (in der Graphitbox) oder unter Schutzgas gesintert wird und anschließend die Abkühlung zwischen 900° C und Raumtemperatur unter einem Schutzgas oder bei Verwendung einer Graphitbox auch unter einem Vakuum von 50 bis 1 HPa erfolgt.

2. Verfahren zur Herstellung von Zahnersatz nach Anspruch 1,
dadurch gekennzeichnet,
daß die Wärmebehandlung zwischen 100° C und 400° C 5 bis 25 min. und das Sintern 10 bis 30 min durchgeführt wird.

3. Verfahren zur Herstellung von Zahnersatz nach Anspruch 1 und 2,
dadurch gekennzeichnet,
daß das Aufheizen auf Sintertemperatur, das Sintern und die Abkühlung unter Schutzgas durchgeführt wird wobei der Sauerstoffpartialdruck im Ofenraum kleiner 5 x 10$^{-2}$ HPa betragen muß.

**Claims**

1. A process for the production of a sintered denture comprising a metallic structural matrix consisting of a noble metal- or noble metal alloy powder mixture having a bimodal or multimodal particle size distribution and predominantly spherical form, which mixture is stirred with a mixing liquid consisting essentially of water to form a slip which can be modelled and can be densified by expulsion of the mixing liquid, by means of which the denture is modelled, using customary techniques for dental ceramics, on a cast, which serves as a firing support, of the teeth which are to be provided, and is then sintered on the cast in a graphite box or in a protective gas atmosphere, characterised in that the denture, having been modelled and initially dried in air for 5 to 25 minutes, is heat-treated for 5 to 45 minutes at a temperature of between 100°C and 400°C, is then heated to 800°C with a mean temperature increase of 50 to 300 K/min, and above 800°C is brought, with a mean temperature increase of 20 to 200 K/min, in air (in the graphite box) or in a protective gas atmosphere, to the sintering temperature T, which is between ($T_{solidus}$ - 200°C) and ($T_{solidus}$ -70°C), $T_{solidus}$ being the solidus temperature of the sintered alloy, and is sintered at this temperature for 5 to 45 minutes in air (in the graphite box) or in a protective gas atmosphere, whereupon cooling is carried out at between 900°C and room temperature in a protective gas atmosphere or, when a graphite box is used, also in a vacuum of 50 to 1 Hpa.

2. A process for the production of a denture as claimed in Claim 1, characterised in that the heat treatment is carried out at between 100°C and 400°C for 5 to 25 minutes and sintering is carried out for 10 to 30 minutes.

3. A process for the production of a denture as claimed in Claims 1 and 2, characterised in that heating to the sintering temperature, sintering and cooling are carried out in a protective gas atmosphere, where the oxygen partial pressure in the furnace chamber must be less than 5 x 10$^{-2}$ HPa.

8

**Revendications**

1. Procédé pour la fabrication d'une prothèse dentaire frittée avec une matrice de structure métallique composé d'un mélange de poudres de métaux nobles ou d'alliages de métaux nobles à distribution bimodale ou multimodale de tailles de particules et forme principalement sphérique, qui est délayé avec un liquide de mélange essentiellement constitué d'eau, pour donner une barbotine pouvant être modelée et épaissie par expulsion du liquide de mélange, avec laquelle la prothèse dentaire est modelée, par la technique usuelle de céramique dentaire, sur un modèle des dents à fournir, servant de support pour la cuisson, et ensuite frittée sur le modèle dans une boîte en graphite ("graphitbox") ou sous un gaz protecteur, procédé caractérisé en ce que la prothèse dentaire modelée et tout d'abord séchée à l'air pendant 5 à 25 minutes est traitée thermiquement entre 100 et 400° C, puis chauffée à 800° C avec une élévation moyenne de température de 50 à 300 K/minute et portée à la température de frittage T supérieure à 800°C avec une élévation moyenne de température de 20 à 200 K/minute à l'air (dans la boîte en graphite) ou sous un gaz protecteur, température T qui est comprise entre ($T_{solidification}$ - 200° C) et ($T_{solidification}$ - 70° C), $T_{solidification}$ étant la température de solidification de l'alliage fritté, puis frittée pendant 5 à 45 minutes à l'air (dans la boîte de graphite) ou sous gaz protecteur, et ensuite le refroidissement est effectué entre 900° C et la température ambiante, sous un gaz protecteur ou avec utilisation d'une boîte en graphite, également sous un vide de 50 à 1 HPa.

2. Procédé pour la fabrication d'une prothèse dentaire selon la revendication 1, caractérisé en ce que le traitement thermique est effectué entre 100 et 400° C pendant 5 à 25 minutes et le frittage est effectué pendant 10 à 30 minutes.

3. Procédé pour la fabrication d'une prothèse dentaire selon la revendication 1 ou 2, caractérisé en ce que le chauffage à la température de frittage, le frittage et le refroidissement sont effectués sous gaz protecteur, la pression partielle d'oxygène dans l'espace du four devant être inférieure à 5 x $10^{-2}$ HPa.

Fig.

EP 0 373 380 B1